# FASCICULE DE BREVET EUROPEEN

(11) **EP 3 416 652 B1**
(45) Date de publication et mention de la délivrance du brevet: **22.07.2020**
(21) Numéro de dépôt: 16810443.8
(22) Date de dépôt: 23.11.2016
(51) Int. Cl.: A61K 31/7016, A61K 31/728, A61P 19/02

(54) **COMPOSITION INJECTABLE; PROCÉDÉ DE PRÉPARATION DE LADITE COMPOSITION; UTILISATION DE LADITE COMPOSITION**
INJIZIERBARE ZUSAMMENSETZUNG, VERFAHREN ZUR HERSTELLUNG DER ZUSAMMENSETZUNG UND VERWENDUNG DER ZUSAMMENSETZUNG
INJECTABLE COMPOSITION, METHOD FOR PREPARING SAID COMPOSITION, AND USE OF SAID COMPOSITION

(30) Priorité: 15.02.2016 FR 1600259
(43) Date de publication de la demande: 26.12.2018
(73) Titulaire: KH Medtech Sàrl, 1204 Genève (CH)
(72) Inventeur: TAUZIN, Bénédicte Vincente, 74250 Bogeve (FR)
(74) Mandataire: reuteler & cie SA
(86) Numéro de dépôt international: PCT/FR2016/000187
(87) Numéro de publication internationale: WO 2017/140958

(56) Documents cités:
- WO-A1-2014/110454
- WO-A2-2012/048854
- WO-A2-2013/186493

## Description

La présente invention est défini par les revendications jointes ci-après. La présente invention a pour objet :
- Une nouvelle composition aqueuse stérile injectable, stérilisée à la chaleur, comprenant au moins de l'acide hyaluronique, ou l'un de ses sels, et un ou plusieurs oses non réducteurs
- Un procédé de préparation de ladite composition
- L'utilisation de ladite composition pour des applications esthétiques et thérapeutiques

La matrice extracellulaire, également appelée ciment intercellulaire, désigne chez les êtres vivants l'ensemble des macromolécules extracellulaires du tissu conjonctif et des autres tissus. Elle est constituée en grande partie de glycoprotéines (collagène, fibronectine et laminine), de protéines (élastine) et de glycosaminoglycanes (dont l'acide hyaluronique est le plus connu).

Le collagène est une famille de protéines, le plus souvent présente sous forme fibrillaire, ayant notamment pour fonction de conférer aux tissus une résistance mécanique à l'étirement. Il est largement présent dans la matrice extracellulaire des organismes vivants humains et animaux : c'est d'ailleurs la famille de protéines la plus abondante chez l'être humain. Le collagène est sécrété par les cellules des tissus conjonctifs : les fibroblastes, les kératinocytes, les chondrocytes, les ostéoblastes et les cellules endothéliales.

L'élastine est une macromolécule de la famille des protéines fibreuses de type structural possédant des propriétés élastiques. Elle est sécrétée dans la peau par les fibroblastes.

L'acide hyaluronique est un polysaccharide formé par la répétition d'une unité disaccharidique composée d'acide D-glucuronique et de N-acétylglucosamine. Sa structure est linéaire et sans spécificité d'espèce. L'acide hyaluronique est largement distribué dans les organismes vivants humains et animaux, dans lesquels il joue de nombreuses fonctions biologiques comme par exemple le contrôle du taux d'hydratation ou le maintien de la viscoélasticité de fluides ou de tissus. On le retrouve notamment en concentration élevée dans le liquide synovial, le corps vitreux de l'œil et dans le derme. Un être humain de 70 kg possède environ 15 g d'acide hyaluronique dont la moitié est contenue dans la peau et cette quantité diminue avec le vieillissement. Dans la peau, l'acide hyaluronique est synthétisé par les fibroblastes et les kératinocytes.

Ces macromolécules clés de la matrice extracellulaire, que sont le collagène, l'élastine et l'acide hyaluronique, sont donc toutes produites par les cellules qui sont à leur contact dans les tissus; cellules (fibroblastes, kératinocytes, chondrocytes, ostéoblastes, ...) spécialisées dans la synthèse de ces différents constituants. La « centrale énergétique » de ces cellules s'appelle la mitochondrie. Son rôle physiologique est primordial, puisque c'est dans les mitochondries que l'énergie fournie par les molécules organiques (issues de la digestion d'oses) est récupérée sous forme d'ATP, la source principale d'énergie pour les cellules eucaryotes, par le processus d'oxydation phosphorylante.

Avec l'âge, l'accumulation des dommages oxydatifs sur les enzymes est responsable d'une diminution de l'efficacité des mitochondries qui sont plus pauvrement équipées en antioxydants et en systèmes de réparation de l'ADN.

Le vieillissement (ou sénescence) est un processus physiologique attribué à la lente dégradation des fonctions de l'organisme. Il est déterminé génétiquement et il est d'une grande variabilité inter-individuelle. En général, le vieillissement cutané s'installe à partir de 25-30 ans mais il s'accélère et devient manifeste vers l'âge de 50 ans. On parle de vieillissement intrinsèque.

De nombreux facteurs extrinsèques peuvent également être responsables d'un vieillissement précoce. On peut évoquer le tabac, les carences nutritionnelles, les maladies et les agressions environnementales comme le soleil, la pollution, le vent, le froid et la sécheresse.

Ces facteurs intrinsèques et extrinsèques agissent avec le même impact négatif final sur les cellules de la peau : les kératinocytes et les fibroblastes ne peuvent plus se développer parfaitement et ainsi ils ne synthétisent plus aussi bien la matrice extracellulaire, milieu essentiel à la vie : moins de collagène, d'élastine et d'acide hyaluronique donnent un aspect moins tendu à la peau.

Au niveau du derme par exemple, les fibroblastes diminuent en nombre et en volume et leur capacité de synthèse protéique est détériorée. Les faisceaux de fibres de collagène se désorganisent et les fibres d'élastine s'amincissent et se raréfient. Le derme perd les capacités de résistance et d'élasticité qu'il offrait à la peau, ce qui induit une apparition de rides et des dommages sur la fermeté et la tonicité cutanée. D'autre part, la qualité et la quantité de protéoglycanes diminuent, ce qui amoindri les fonctions d'hydratation cutanée, provoquant ainsi un desséchement de la peau.

Ainsi, le vieillissement est lié à une altération des mécanismes de réparation et/ou de maintenance des cellules. Il est déterminé, d'une part, par une sorte d'horloge biologique individuelle génétiquement programmée, et d'autre part, par les capacités de résistance des cellules aux dégâts oxydatifs causés par des substances délétères, les radicaux libres.

L'agression des cellules par les radicaux libres entraîne des modifications au niveau des mitochondries, notamment une perte d'efficacité comme décrit précédemment, engendrant une diminution de la production de l'énergie vitale pour les cellules.

Dans ce contexte, la présente invention concerne, selon un premier de ses aspects, une nouvelle composition aqueuse stérile injectable, stérilisée à la chaleur, sous forme de gel, comprenant au moins de l'acide hyaluronique réticulé ou non réticulé, ou l'un de ses sels, et du tréhalose, à « l'état libre », c'est-à-dire simplement dispersés (et non réticulés/greffés) au sein du gel d'acide hyaluronique.

Cette nouvelle composition a pour objectif principal les deux points suivants ayant un bénéfice direct sur l'efficacité clinique d'un traitement par injection dans les tissus mous, et en particulier dans les tissus de la peau :
- Stimuler la production d'énergie par les mitochondries des cellules sénescentes, ceci afin qu'elles accroissent leur activité de synthèse de collagène, d'élastine et d'acide hyaluronique, permettant ainsi de redonner, dans le cas du traitement de la peau, de la souplesse, de la résistance, de l'élasticité et de l'hydratation cutanée, limitant ainsi les signes visibles de vieillissement tels que les rides ou le dessèchement de la peau
- Compenser directement les effets négatifs liés à la diminution de la production de collagène, d'élastine et d'acide hyaluronique en comblant le manque de ces molécules par l'administration d'un gel aux propriétés biomécaniques de viscoélasticité adaptées à la matrice extracellulaire, et permettant par exemple dans le cas du traitement de la peau, de combler des rides ou de restaurer/augmenter des volumes au niveau du visage ou du corps

Cette composition est constituée d'une combinaison de deux ingrédients clés, l'acide hyaluronique réticulé ou non réticulé, ou l'un de ses sels, et du tréhalose. De manière très intéressante et surprenante, ces deux ingrédients, de nature chimique similaire, possèdent une forte affinité l'un pour l'autre, sans pour autant être en mesure de réagir entre eux, et ceci, sans vouloir se lier à un explication, du fait de la grande stabilité et de l'inertie des oses non réducteurs simples qui ne sont pas susceptibles de participer aux réactions de Maillard (contrairement aux oses réducteurs simples) empêchant ainsi le développement de composés indésirables (au cours du procédé de fabrication et/ou au cours de la durée de péremption du produit selon l'invention) pouvant être colorés, odorants et potentiellement toxiques.

Dans cette composition, l'acide hyaluronique permet de supplémenter les tissus de la peau (ou les autres tissus visés) par injection notamment dans la matrice extracellulaire dermique ou sous-cutanée, sur une longue période allant de quelques jours à plus de 18 mois. Cette supplémentation par une molécule naturellement présente dans la peau, possédant des propriétés de viscoélasticité et de longévité (c'est-à-dire de durabilité *in vivo*) remarquables en particulier lorsqu'elle est réticulée, permet d'apporter un bénéfice clinique immédiat au patient traité en améliorant la qualité de la peau et/ou en réduisant les rides et/ou en restaurant/augmentant les volumes du visage ou du corps.

Le tréhalose permet aux cellules sénescentes du derme et des tissus sous-cutanés (en particulier les fibroblastes) de stimuler la production endogène de collagène, d'élastine et d'acide hyaluronique en leur apportant l'énergie nécessaire pour produire à nouveau ou pour produire davantage.

Une fois injectée dans les tissus, la composition selon l'invention libère le tréhalose au niveau de la zone traitée, au cours du temps ce dernier, du fait de son caractère non réducteur et non modifié, ne réagira pas avec les protéines ou acides aminés environnants dans le milieu extracellulaire, tout comme dans le milieu intracellulaire (via des réactions de Maillard), conservant ainsi toute la nature et la fonctionnalité de ces molécules d'intérêt biologique. De ce fait, il est primordial de noter que les oses non réducteurs simples (contrairement aux oses réducteurs simples) présentent un niveau de toxicité négligeable vis-à-vis de l'environnement *in vivo* dans lequel ils sont administrés, et donc permettent de conférer aux gels injectables issus de l'invention un haut niveau de sécurité absolument indispensable pour ce type de produits injectables.

Après libération du tréhalose au niveau de la zone traitée, le mécanisme d'action suivant peut être mis en avant : les oses sont hydrolysés par les enzymes osidases (clivant les liaisons osidiques) qui les dégradent en mono-oses (oses à un cycle) qui correspondent aux substrats énergétiques. Les enzymes hexokinases phosphorylent ensuite ces différents mono-oses sur le carbone numéro 6, permettant alors à ces composés de rejoindre la glycolyse au niveau du fructose après épimérisation ou isomérisation. C'est ainsi que les oses donnent la possibilité de nourrir/alimenter les cellules sénescentes en manque d'énergie, et ainsi stimuler/favoriser la synthèse endogène de collagène, d'élastine et d'acide hyaluronique. Dans les conditions de l'invention, les oses agissent comme un actif « bio-énergisant » pour stimuler les cellules présentes au niveau de la zone traitée.

Il est important de préciser que l'affinité chimique entre l'acide hyaluronique réticulé et le tréhalose dans les conditions de l'invention, peut permettre de viser des cinétiques de libération du tréhalose hors du gel sur des périodes de temps particulièrement longues afin de maximiser la performance long terme du traitement par un produit injectable issu de la présente invention.

La combinaison d'acide hyaluronique réticulé et du tréhalose selon l'invention permet également de bénéficier d'une synergie précieuse vis-à-vis de l'efficacité clinique du produit. En effet, l'acide hyaluronique réticulé lui, permet une libération retardée du tréhalose au niveau de la zone dans laquelle le gel a été injecté (il joue ainsi le rôle de réservoir de molécules à visée énergétique, et il est directement en contact avec les cellules à stimuler, appliquant même une pression sur celles-ci; pression bénéfique à la sécrétion des composés de la matrice extracellulaire par les cellules) alors que le tréhalose est en mesure d'agir au niveau des cellules, pour les renforcer, et notamment pour stimuler leur capacité antioxydante; capacité permettant alors de limiter la dégradation de l'acide hyaluronique administré (et donc la libération du tréhalose qui n'a pas encore été relargué) en neutralisant partiellement ou totalement les radicaux libres (ces entités ayant un fort potentiel de dégradation de l'acide hyaluronique par coupure de chaînes) présents au niveau de la zone traitée. Cette synergie, induite par la composition selon l'invention, génère donc un métabolisme amélioré aux niveaux cellulaires et une durabilité/longévité plus importante de l'acide hyaluronique réticulé afin d'obtenir une efficacité clinique améliorée du produit sur le plus long terme.

Il est également important de préciser que, de façon tout à fait surprenante et inattendue, il a été observé par des tests expérimentaux que la composition selon l'invention possède des propriétés rhéologiques de viscoélasticité améliorées, après stérilisation à la chaleur, par rapport à une composition de l'art antérieur ne contenant pas d'oses non réducteurs simples. En effet, il a été constaté que le module élastique G', bien connu par l'homme de l'art car fournissant une indication fiable sur la capacité du produit à créer du volume en projetant les tissus (par exemple, Sundaram et al., Plast Reconst Surg, 2013, 132:5S-21S), est plus important dans le cas d'un produit selon l'invention. Sans vouloir se lier à une explication, on suppose que le tréhalose joue le rôle de stabilisateur thermique de l'acide hyaluronique réticulé au cours de la stérilisation à la chaleur; stabilisation permettant alors à la composition selon l'invention de bénéficier d'un acide hyaluronique réticulé stérile, stérilisé à la chaleur (donc bénéficiant d'un très haut niveau de stérilité pour une sécurité maximale du dispositif injectable), moins dégradé et donc moins dénaturé que dans le cas d'un produit de l'art antérieur ne contenant pas d'oses non réducteurs simples. Il est à noter que ce même constat n'a pas été observé avec l'utilisation d'un ose réducteur simple, composé bien moins stable qu'un ose non réducteur simple.

Selon un aspect de l'invention, le tréhalose est utilisé en tant qu'agent de réticulation de l'acide hyaluronique réticulé dans la composition selon l'invention. Dans ce cas et exclusivement dans ce cas, les oses non réducteurs ne sont pas des oses non réducteurs simples mais des oses non réducteurs qui ont été modifiés chimiquement ou par toute autre voie préalable pour intégrer des fonctions réactives leur permettant de jouer le rôle de réticulant de l'acide hyaluronique. L'ose non réducteur modifié est alors utilisé/formulé seul en tant que réticulant dans la composition selon l'invention ou alors en combinaison avec des oses non réducteurs simples à « l'état libre », c'est-à-dire simplement dispersés (et non réticulés/greffés) au sein de la matrice d'acide hyaluronique réticulé.

Selon l'invention, l'acide hyaluronique est réticulé. Dans ce cas, le ou les agents réticulants qui interviennent généralement dans la réticulation de l'acide hyaluronique sont des composés bi- ou poly-fonctionnels de différents types et ils peuvent par exemple être sélectionnés parmi la divinylsulfone, les époxy bi- ou poly-fonctionnels, les carbodiimides et le formaldéhyde. Les agents de la famille des époxy bi- ou poly-fonctionnels et notamment le 1,4-butanedioldiglycidyléther (BDDE), le diépoxy-octane ou le 1,2-bis-(2,3-époxypropyl)-2,3-éthylène sont avantageusement choisis.

Dans un mode de réalisation de l'invention, l'agent de réticulation bi ou poly-fonctionnel est un dérivé d'ose non réducteur possédant au moins deux fonctions réactives vis-à-vis des fonctions hydroxyles et carboxylates de l'acide hyaluronique (en milieu aqueux (alcalin ou acide), ou en milieu organique).

Les fonctions réactives greffées aux oses non réducteurs sont choisies parmi les époxydes (préférentiellement glycidyl éther), les halides, les amines, les vinyles, les cétones, les aldéhydes, les thiocyanates, les isocyanates, les halosiliciums, les nitriles ou les sultanes. Ce sont ces fonctions réactives qui transforment l'ose non réducteur en réticulant.

Chaque réticulant à base d'ose non réducteur possède ainsi une fonction réactive à l'extrémité de la chaîne principale d'ose. La fonction réactive (R) peut être fixée préférentiellement sur l'alcool primaire de l'ose (O-ose-O), soit directement soit via un bras espaceur (S), suivant l'enchaînement R-S-O-ose-O-S-R.

De manière alternative, la fonction réactive peut être attachée sur une fonction organique de l'ose autre que l'alcool primaire, via un bras espaceur (c'est-à-dire, dans le cas d'une fonction réactive époxyde, la fonction époxyde ne doit pas être attachée directement sur l'ose).

Dans les deux cas ci-dessus, la longueur du bras espaceur S doit être assez courte, afin d'avoir notamment une toxicité la plus faible possible (on peut citer, par exemple, le méthyl ou l'éthyl glycidyl éther).

La liaison créée entre l'ose et le bras espaceur (ou l'ose et la fonction réactive dans le cas d'une fonction réactive directement greffée sur l'alcool primaire de l'ose) doit être stable en milieu aqueux neutre, acide ou alcalin (par exemple, une liaison éther sera préférée à une liaison ester). De manière avantageuse, le réticulant dérivé d'un ose, pour la réticulation de l'acide hyaluronique réticulé selon un aspect de l'invention, est le Tréhalose DiGlycidyl Ether (TDGE), de formule C₁₈H₃₀O₁₃, caractérisé en ce que deux fonctions réactives glycidyl éther sont greffées sur les alcools primaires du tréhalose par création de liaisons éther.

Selon l'invention, la composition contient de l'acide hyaluronique ou l'un de ses sels, et en particulier ses sels acceptables d'un point de vue physiologique comme les sels de sodium, calcium, zinc, potassium, avantageusement le sel de sodium. L'acide hyaluronique peut être d'origine animale ou obtenu par fermentation bactérienne. Il peut avoir une masse moléculaire de quelques daltons à plusieurs millions de daltons, avantageusement d'environ 0.01 à 5 millions de daltons, encore avantageusement d'environ 0.1 à 3.5 millions de daltons.

Selon un aspect de l'invention, la composition peut être à base d'un dérivé de l'acide hyaluronique, c'est-à-dire à base d'une molécule obtenue en modifiant par voie chimique, ou par toute autre voie, la molécule d'acide hyaluronique.

Selon l'invention, la concentration totale en acide hyaluronique, ou l'un de ses sels, est comprise entre 0.001 et 70 mg/ml, entre 0.01 et 50 mg/ml, entre 1 et 40 mg/ml, entre 5 et 35 mg/ml, entre 8 et 33 mg/ml, entre 9 et 30 mg/ml, entre 10 et 29 mg/ml, entre 11 et 28 mg/ml, entre 12 et 27 mg/ml, entre 13 et 26.5 mg/ml, avantageusement entre 14 et 26 mg/ml.

Selon un aspect avantageux de l'invention, l'acide hyaluronique contenu dans la composition est réticulé, au moins partiellement, et ceci préférentiellement selon les techniques de réticulation décrites dans l'art antérieur. Les températures de réticulation sont généralement comprises entre environ 15°C et 60°C et les durées de réticulation sont généralement de plusieurs heures, avantageusement de plus de 1h jusqu'à environ 24h.

En terme d'agents de réticulation, comme décrit précédemment, l'acide hyaluronique peut être ponté avec les réticulants de l'art antérieur ou, selon un aspect de l'invention, en utilisant un réticulant dérivé d'un ose non réducteur qui a été rendu bi- ou poly-fonctionnel par voie chimique ou par toute autre voie. De manière avantageuse, le réticulant choisi est le BDDE (réticulant de l'art antérieur) ou le tréhalose diglycidyl éther (réticulant selon l'invention).

Selon l'invention, la composition contient une proportion massique en ose non réducteur simple supérieure à 0.001% de la masse totale, supérieure à 0.01% de la masse totale, supérieure à 0.05% de la masse totale, supérieure à 0.1% de la masse totale, supérieure à 0.15% de la masse totale, supérieure à 0.5% de la masse totale, supérieure à 0.75% de la masse totale, supérieure à 1% de la masse totale, supérieure à 2% de la masse totale, supérieure à 3% de la masse totale, supérieure à 4% de la masse totale.

Selon l'invention, la composition contient une proportion massique en ose non réducteur simple inférieure à 20% de la masse totale, inférieure à 10% de la masse totale, inférieure à 8% de la masse totale, inférieure à 6% de la masse totale, inférieure à 5% de la masse totale.

Selon un aspect de l'invention, le ratio massique entre la concentration massique en acide hyaluronique [HA] et la concentration massique en oses non réducteurs simples [Ose NR], c'est-à-dire le ratio [HA]/[Ose NR], est compris entre 0.01 et 250

Selon l'invention, la composition contient du tréhalose

Il est important de noter que le sucrose (un ose non réducteur simple possédant 2 unités saccharidiques) n'est pas un ose acceptable selon l'invention. En effet, ce disaccharide joue un rôle central dans le diabète, maladie touchant des millions de personnes à travers le monde, et de ce fait, il n'est pas compatible avec le but premier de cette invention qui est de mettre à la disposition du plus grand nombre, une composition injectable à haut niveau de sécurité et performance.

Selon un aspect de l'invention, un ou plusieurs oses non réducteurs modifiés par ajout de fonctions réactives peuvent être utilisés au sein de la composition exclusivement en tant que réticulant de l'acide hyaluronique. Par oses non réducteurs modifiés par ajout de fonctions réactives, on entend les oses non réducteurs qui ont été transformés chimiquement ou par toute autre voie au préalable pour intégrer des fonctions réactives leur permettant de jouer le rôle de réticulant de l'acide hyaluronique, comme décrit précédemment et illustré par l'exemple avec le Tréhalose DiGlycidyl Ether (TDGE).

Selon l'invention, la composition a pour ingrédient majoritaire l'eau (en proportion massique), d'où le qualificatif de composition aqueuse pour la nouvelle composition selon l'invention. La masse en eau dans la composition selon l'invention est supérieure à 51% de la masse totale, avantageusement supérieure à 60% de la masse totale, avantageusement supérieure à 70% de la masse totale, avantageusement supérieure à 75% de la masse totale, avantageusement supérieure à 80% de la masse totale, avantageusement supérieure à 85% de la masse totale. Une solution tampon est avantageusement utilisée notamment pour mieux contrôler le pH et l'osmolarité de la formulation tout au long de sa durée de péremption. On peut par exemple citer l'utilisation d'un tampon à base de chlorure de sodium et d'ions phosphates.

Selon l'invention, la composition est stérile. Elle est stérilisée selon les techniques décrites dans l'art antérieur. Elle est avantageusement stérilisée à la chaleur, préférentiellement à la chaleur humide (également appelée autoclavage à la vapeur).

De manière préférée, la stérilisation à la chaleur humide est effectuée à une température supérieure à 100°C, avantageusement supérieure à 110°C, avantageusement supérieure à 120°C. De manière générale, la durée de stérilisation peut aller de quelques secondes à plusieurs minutes. On peut citer par exemple les cycles de stérilisation à la chaleur humide suivants : 121°C pendant 20 minutes ou 125°C pendant 7 minutes ou 127°C pendant 4 minutes ou encore 130°C pendant 3 minutes.

Il est important de noter que la stérilisation à la chaleur est avantageusement sélectionnée car elle octroie notamment un très haut niveau de stérilité à la composition choisie, ce qui permet alors de viser un haut niveau de sécurité pour le patient traité.

Selon l'invention, la composition est injectable. Elle est préférentiellement conditionnée dans une seringue ou dans un vial, afin de pouvoir être facilement administrée à travers une aiguille ou une canule.

Selon l'invention, les molécules d'oses non réducteurs simples sont dispersées dans l'hydrogel de manière homogène ou relativement homogène.

Selon un aspect de l'invention, la composition selon l'invention contient une ou plusieurs substances actives d'origine naturelle ou synthétique à action pharmacologique ou non, comme par exemple les antioxydants, les anti-inflammatoires, les antiseptiques, les antibactériens, les antifongiques, les anticancéreux, les anesthésiques locaux, les protéines, les hormones, seuls ou en combinaison. Ces substances actives sont soit dispersées dans l'hydrogel, soit greffées à un ou plusieurs des polymères de l'hydrogel, soit contenues/encapsulées dans des liposomes/niosomes dispersés dans l'hydrogel, soit contenues/encapsulées dans un autre matériau lui-même dispersé au sein de l'hydrogel.

Selon un aspect avantageux de l'invention, la composition selon l'invention contient de la lidocaïne (ou tout autre anesthésique) dispersée au sein de l'hydrogel.

Selon un aspect de l'invention, la composition selon l'invention contient un ou plusieurs composés d'origine biologique comme des cellules, des plaquettes enrichies, des gènes, des fragments d'ADN ou des facteurs de croissance. Ces composés sont préférentiellement dispersés dans l'hydrogel, mais ils peuvent également être greffés à un ou plusieurs des polymères de l'hydrogel ou contenus/encapsulés dans des liposomes/niosomes dispersés dans l'hydrogel ou contenus/encapsulés dans un autre matériau lui-même dispersé au sein de l'hydrogel.

Selon un aspect de l'invention, la composition selon l'invention contient des polymères qui sont dispersés au sein de la matrice réticulée de l'hydrogel. On peut citer par exemple les polymères de la famille des polysaccharides (et notamment de l'acide hyaluronique non réticulé), les polyesters, les polyanhydrides, les polyphosphazenes, les poly-ε-caprolactones, les acides polylactiques et leurs dérivés, les acides polyvinyliques, les polyacrylamides, la N-vinyl pyrrolidone et les polymères acryliques et dérivés biologiquement acceptables.

Selon un aspect de l'invention, la composition selon l'invention contient des substances minérales qui sont dispersées au sein de la matrice réticulée de l'hydrogel. On peut citer par exemple l'hydroxyapatite ou les phosphates tricalciques comme le β tricalcium phosphate.

La présente invention concerne, selon un deuxième de ses aspects, un procédé de préparation de la nouvelle composition aqueuse stérile injectable décrite précédemment.

Le procédé de préparation de la composition selon l'invention se caractérise par les étapes successives suivantes :
a) préparation d'un gel à base d'acide hyaluronique réticulé ou l'un de ses sels
b) ajout du ou des oses non réducteurs simples dans le gel avec opération de mélange
c) stérilisation

Dans le cas plus complexe de la présence d'acide hyaluronique réticulé dans la composition, les détails suivants peuvent être fournis pour la réalisation de l'étape (a).

L'étape (a) débute généralement par la mise en solution de l'acide hyaluronique puis par sa réticulation, à l'aide d'un réticulant, et elle se termine généralement par la purification de l'hydrogel obtenu.

On entend par étape de réticulation de l'acide hyaluronique, l'étape permettant de ponter les chaînes d'acide hyaluronique les unes aux autres par des liaisons covalentes. De manière générale, l'étape de réticulation de l'acide hyaluronique commence lorsque le réticulant est mis en contact avec l'acide hyaluronique et se termine lorsque l'homme de l'art considère que la cinétique de réaction de pontage des chaînes d'acide hyaluronique par le réticulant a atteint un niveau négligeable.

La purification de l'hydrogel, elle, permet de retirer les molécules indésirables du gel préparé et en particulier les résidus de réticulant, suite à la réticulation. Cette purification est effectuée selon les techniques bien connues par l'homme de l'art comme par exemple par bains de dialyse, par lavage par flux d'eau continu ou par précipitation.

La préparation d'un hydrogel à base d'acide hyaluronique réticulé est effectuée avantageusement selon les méthodes décrites dans l'art antérieur. On pourra par exemple citer les demandes WO 97/04012, WO 2004/092222, WO 2005/085329 et WO 2009/071697 pour la fabrication d'un hydrogel à base d'acide hyaluronique réticulé.

L'étape (b) consiste à ajouter un ou plusieurs oses non réducteurs simples dans le gel à base d'acide hyaluronique préalablement préparé.

Cet ajout du ou des oses non réducteurs simples s'accompagne d'une opération de mélange ayant vocation à homogénéiser le ou les oses non réducteurs simples au sein de l'hydrogel. Le mélange peut se faire pendant l'ajout et/ou après l'ajout.

L'opération de mélange est effectuée par les techniques bien connues par l'homme de l'art comme par exemple par mélange mécanique au sein d'une cuve de mélange.

Le mélange peut être effectué à une température supérieure à la température ambiante et/ou sous pression et/ou sous ultra-sons afin d'optimiser l'homogénéisation de la composition.

L'étape (c) consiste à stériliser la composition par les techniques bien connues par l'homme de l'art; la stérilisation étant avantageusement réalisée à la chaleur par autoclavage.

Dans le cas d'une composition aqueuse stérile injectable selon l'invention contenant de l'acide hyaluronique réticulé, un procédé avantageux selon l'invention pour la fabrication de cette composition comprend au moins les étapes suivantes :
- préparation d'une solution aqueuse d'acide hyaluronique
- réticulation de l'acide hyaluronique
- purification de l'hydrogel, par exemple par dialyse dans une solution physiologique
- ajout d'un ou plusieurs oses non réducteurs simples avec opération de mélange
- conditionnement en seringues
- stérilisation

Un autre procédé avantageux selon l'invention pour la fabrication de cette composition aqueuse stérile injectable selon l'invention comprend au moins les étapes suivantes :
- préparation d'une solution aqueuse d'acide hyaluronique
- réticulation de l'acide hyaluronique
- purification de l'hydrogel, par exemple par dialyse dans une solution contenant un ou plusieurs oses non réducteurs simples suivi par une opération de mélange
- conditionnement en seringues
- stérilisation

Selon un aspect de l'invention, comme décrit précédemment, la réticulation de l'acide hyaluronique peut être réalisée en utilisant un réticulant dérivé d'un ose non réducteur selon l'invention au lieu d'un réticulant de l'art antérieur.

Dans ce cas, le procédé de préparation de la composition selon l'invention se caractérise par les étapes successives suivantes :
- préparation d'un gel à base d'acide hyaluronique réticulé, ou l'un de ses sels, en utilisant un dérivé d'un ose non réducteur transformé au préalable en composé bi- ou poly-fonctionnel par voie chimique ou par toute autre voie, en tant qu'agent de réticulation
- ajout optionnel d'un ou de plusieurs oses non réducteurs simples dans le gel avec opération de mélange
- stérilisation

La présente invention concerne, selon un troisième de ses aspects, l'utilisation chez l'homme ou chez l'animal de la nouvelle composition aqueuse stérile injectable décrite précédemment, pour des applications esthétiques ou thérapeutiques.

La composition aqueuse stérile injectable selon l'invention est notamment utilisée pour :
- combler des volumes
- générer des espaces au sein de certains tissus, favorisant ainsi leur fonctionnement optimal
- remplacer des liquides physiologiques ou des tissus déficients
- stimuler ou favoriser la régénération des tissus
- hydrater et protéger des tissus
- délivrer des substances susceptibles d'apporter un bénéfice à l'organisme et notamment des substances actives et/ou des biologiques

A titre d'exemple, on peut citer les utilisations de l'hydrogel dans les cas suivants :
- la formulation d'une composition injectable en intradermique ou en sous-cutané pour l'amélioration de la qualité de la peau ou le comblement des rides ou la restauration des volumes du visage (pommettes, menton, lèvres, nez, ...) ou du corps
- la formulation d'une composition injectable à usage dentaire par exemple pour combler des poches parodontales et/ou pour stimuler la régénération des tissus autour de la dent
- la formulation d'une composition injectable en intra-oculaire, notamment pour des applications au cours de la chirurgie de la cataracte, du glaucome, de la presbytie ou du vitré
- la formulation d'une composition injectable en intra-articulaire pour des applications en orthopédie ou en rhumatologie, notamment dans le cadre de la viscosupplémentation du liquide synovial déficient pour le traitement de l'arthrose mais également de la reconstruction osseuse ou de la régénération du cartilage
- la formulation d'une composition injectable en urologie pour des applications dans le traitement de l'incontinence urinaire ou fécale
- la formulation d'une composition injectable utilisée en médecine ou chirurgie générale dans le cadre du traitement de la fibrose ou pour améliorer la cicatrisation des plaies
- la formulation d'une composition pharmaceutique injectable permettant la libération contrôlée de substances actives et/ou de biologiques pour différentes applications médicales

### EXEMPLES

L'invention va maintenant être illustrée, par les exemples suivants.

Le hyaluronate de sodium, les oses et l'ensemble des autres composés utilisés dans les exemples suivants possèdent un haut niveau de pureté.

Les propriétés rhéologiques (mesure du module élastique G' à 0.7 Hz) des gels sont mesurées à 25°C à l'aide d'un rhéomètre à contrainte imposée (TA AR2000) et d'une géométrie cône/plan 4 cm-2° avec un entrefer de 1000 micromètres.

### Exemple 1 : Préparation d'un gel G1 selon l'invention et des gels X1 et REF1 (comparatif)

On pèse 2.5 g d'une poudre de hyaluronate de sodium (NaHA), de masse moléculaire environ égale à 1.8 MDa, avec un taux d'humidité de 5.2%, auxquels on ajoute 21 g d'une solution aqueuse de NaOH à 0.25 N. L'hydratation de la poudre dure 1h30, avec une homogénéisation manuelle régulière à la spatule. 1.1 g d'une solution de 1,4-butanedioldiglycidyléther (BDDE) dilué au 1/5^{ème} dans la soude 0.25 N sont ajoutés au milieu réactionnel, suivi d'une homogénéisation mécanique de 15 minutes avant immersion dans un bain thermostaté à 50°C pendant 2h30. On ajoute une solution de tampon phosphate contenant du HCl dans le réticulat obtenu afin d'obtenir un pH=7.3 et une concentration en acide hyaluronique égale à 36 mg/g. On laisse le gel gonfler pendant 24h à température ambiante dans cette solution et, à la fin de ce temps, on l'homogénéise manuellement à la spatule pendant 10 minutes avant de le purifier par dialyse pendant 24h en utilisant une membrane à base de cellulose (seuil de rétention = 10 000 Da) dans une solution de tampon phosphate. On mélange manuellement le gel à la spatule pendant 10 minutes. Soit REF1A le gel ainsi obtenu. La concentration en acide hyaluronique de REF1A est de 27 mg/g.

On partage ensuite le gel REF1A en 3 fractions de masses égales.

Dans la fraction 1, on ajoute du tréhalose (TH), un ose non réducteur simple.

Pour cela, on prépare une solution aqueuse de tréhalose à 120 mg/g dans une solution de tampon phosphate. On dilue ensuite la fraction 1 du gel REF1A afin d'obtenir un gel avec une concentration en acide hyaluronique de 20 mg/g et une concentration en tréhalose de 30 mg/g (ratio massique [HA]/[TH] égal à 0.67). On homogénéise ensuite pendant 10 minutes à la spatule. Soit G1A le gel ainsi obtenu.

Dans la fraction 2, on ajoute du lactose (L), un ose réducteur simple.

Pour cela, on prépare une solution de lactose à 120 mg/g dans une solution de tampon phosphate. On dilue ensuite la fraction 2 du gel REF1A afin d'obtenir un gel avec une concentration en acide hyaluronique de 20 mg/g et une concentration en lactose de 30 mg/g (ratio massique [HA]/[L] égal à 0.67). On homogénéise ensuite pendant 10 minutes à la spatule. Soit X1A le gel ainsi obtenu.

Dans la fraction 3, on ajoute une solution de tampon phosphate afin d'obtenir une concentration en acide hyaluronique de 20 mg/g. On homogénéise ensuite pendant 10 minutes à la spatule. Soit REF1AA le gel ainsi obtenu.

Les gels G1A, X1A et REF1AA sont conditionnés en seringues de 1 ml puis stérilisés à l'autoclave à 121°C pendant 18 minutes. Soit G1 (= gel selon l'invention), X1 et REF1 les gels stérilisés issus des gels G1A, X1A et REF1AA.

La nouvelle composition G1 selon l'invention, stérile, stérilisée à la chaleur, est facilement injectable à travers une aiguille fine de 27G ½, la rendant tout à fait compatible avec des injections dans les tissus mous d'un organisme vivant animal ou humain pour des applications esthétiques ou médicales.

### Exemple 2 : Préparation des gels G2, G3 et G4 selon l'invention

On prépare un gel REF1A selon le même mode opératoire que celui décrit dans l'exemple 1.

On partage ensuite le gel REF1A en 3 fractions de masses égales.

Dans la fraction 1, on ajoute du tréhalose (TH), un ose non réducteur simple, et du chlorhydrate de lidocaïne.

Pour cela, on prépare une solution de tréhalose à 40 mg/g dans une solution de tampon phosphate. On dilue ensuite la fraction 1 du gel REF1A afin d'obtenir un gel avec une concentration en acide hyaluronique de 20 mg/g et une concentration en tréhalose de 2 mg/g (ratio massique [HA]/[TH] égal à 10). On ajoute ensuite 3 mg/g d'une poudre de chlorhydrate de-lidocaïne et on homogénéise pendant 10 minutes à la spatule. Soit G2A le gel ainsi obtenu.

Dans la fraction 2, on ajoute du stachyose (SC), un ose non réducteur simple, et du mannitol.

Pour cela, on prépare une solution de stachyose à 40 mg/g dans une solution de tampon phosphate. On dilue ensuite la fraction 2 du gel REF1A afin d'obtenir un gel avec une concentration en acide hyaluronique de 20 mg/g et une concentration en stachyose de 2 mg/g (ratio massique [HA]/[SC] égal à 10). On ajoute ensuite 30 mg/g d'une poudre de mannitol et on homogénéise pendant 10 minutes à la spatule. Soit G3A le gel ainsi obtenu.

Dans la fraction 3, on ajoute du tréhalose (TH), un ose non réducteur simple.

Pour cela, on prépare une solution de tréhalose à 670 mg/g dans une solution de tampon phosphate. On dilue ensuite la fraction 3 du gel REF1A afin d'obtenir un gel avec une concentration en acide hyaluronique de 5 mg/g et une concentration en tréhalose de 500 mg/g (ratio massique [HA]/[TH] égal à 0.01). Soit G4A le gel ainsi obtenu.

Les gels G2A, G3A et G4A sont conditionnés en seringues de 1 ml puis stérilisés à l'autoclave à 131°C pendant 8 minutes. Soit G2, G3 et G4 (= 3 gels selon l'invention), les gels stérilisés issus des gels G2A, G3A et G4A.

### Exemple 3 : Préparation des gels G5A et G5B selon l'invention

On prépare un gel selon le même mode opératoire que celui décrit dans l'exemple 1, mais on remplace le réticulant BDDE de l'art antérieur par un nouveau réticulant selon l'invention à base d'ose non réducteur, le Tréhalose DiGlycidyl Ether (TDGE).

Le TDGE est synthétisé via une allylation par voie directe du tréhalose.

Soit REF5A le gel ainsi obtenu. La concentration en acide hyaluronique de REF5A est de 27 mg/ml.

On partage ensuite le gel REF5A en 2 fractions de masses égales.

Dans la fraction 1, on ajoute du tréhalose (TH), un ose non réducteur simple.

Pour cela, on prépare une solution aqueuse de tréhalose à 120 mg/ml dans une solution de tampon phosphate. On dilue ensuite la fraction 1 du gel REF5A afin d'obtenir un gel avec une concentration en acide hyaluronique de 20 mg/g et une concentration en tréhalose de 30 mg/g (ratio massique [HA]/[TH] égal à 0.67). On homogénéise ensuite pendant 10 minutes à la spatule. Soit G5AA le gel ainsi obtenu.

Dans la fraction 2, on ajoute une solution de tampon phosphate afin d'obtenir une concentration en acide hyaluronique de 20 mg/g, après homogénéisation manuelle de 10 minutes à la spatule. Soit G5BB le gel ainsi obtenu.

Les gels G5AA et G5BB sont conditionnés en seringues de 1 ml puis stérilisés à l'autoclave à 121°C pendant 18 minutes. Soit G5A et G5B (= 2 gels selon l'invention), les gels stérilisés issus des gels G5AA et G5BB.

### Exemple 4 : Caractérisation des propriétés rhéologiques des produits de l'exemple 1

Les propriétés rhéologiques de viscoélasticité (mesure du module élastique G' à 0.7 Hz) sont mesurées (6 mesures par gel dont la moyenne est présentée dans les tableaux ci-dessous) pour les gels de l'exemple 1 avant stérilisation et après stérilisation à l'autoclave.

### Avant stérilisation à l'autoclave :

| Gel | Description | G' à 0.7 Hz (Pa) |
|---|---|---|
| G1A | NaHA réticulé à 20 mg/ml + tréhalose à 30 mg/ml (ose non réducteur simple) - non stérile | 252 |
| X1A | NaHA réticulé à 20 mg/ml + lactose à 30 mg/ml (ose réducteur simple) - non stérile | 256 |
| REF1AA | NaHA réticulé à 20 mg/ml - non stérile | 249 |

Avant stérilisation, on constate que les gels G1A, X1A et REF1AA ont des modules élastiques similaires.

### Après stérilisation à l'autoclave :

| Gel | Description | G' à 0.7 Hz (Pa) |
|---|---|---|
| G1 (selon l'invention) | NaHA réticulé à 20 mg/ml + tréhalose à 30 mg/ml (ose non réducteur simple) - stérile | 179 |
| X1 | NaHA réticulé à 20 mg/ml + lactose à 30 mg/ml (ose réducteur simple) - stérile | 141 |
| REF1 | NaHA réticulé à 20 mg/ml - stérile | 156 |

Après stérilisation à la chaleur (stérilisation permettant d'atteindre un haut niveau de stérilité pour assurer un très haut niveau de sécurité du dispositif injectable), on constate de manière intéressante et surprenante que le produit G1 selon l'invention possède un module élastique G' significativement plus élevé que pour le produit REF1 de l'art antérieur (ne contenant pas d'ose) et que le produit X1 qui lui contient un ose réducteur simple. On constate d'ailleurs pour ce dernier (produit X1 avec l'ose réducteur simple) que le module élastique G' mesuré est inférieur à celui du produit référence REF1 de l'art antérieur ne contenant pas d'ose.

### Détermination du pourcentage de perte du module élastique G' lors de la stérilisation à l'autoclave :

| Gel | Description | Variation de G' à la stérilisation |
|---|---|---|
| G1 (selon l'invention) | NaHA réticulé à 20 mg/ml + tréhalose à 30 mg/ml (ose non réducteur simple) - stérile | -29% |
| X1 | NaHA réticulé à 20 mg/ml + lactose à 30 mg/ml (ose réducteur simple) - stérile | -45% |
| REF1 | NaHA réticulé à 20 mg/ml - stérile | -37% |

Pour l'ensemble des produits testés, on observe une diminution du module élastique G' à la stérilisation. Cette observation est en parfaite corrélation avec la littérature qui décrit que l'acide hyaluronique est sensible à la température et que, par conséquent, il se dégrade par coupure de chaînes au cours d'un événement thermique.

Par contre, on constate que le pourcentage de diminution du module élastique G' est significativement inférieur pour le produit G1 selon l'invention par rapport aux autres produits REF1 et X1.

Cela confère un avantage clinique conséquent en termes de sécurité et performance au produit selon l'invention, en parallèle de l'ensemble des autres bénéfices clés décrit précédemment. En effet, cela permet de viser des modules élastiques G' plus élevés et donc une meilleure capacité à créer du volume dans les tissus en les projetant/poussant davantage (ce qui est clé dans le domaine de l'esthétique, par exemple pour formuler des produits injectables dédiés à la restauration des volumes du visage ou en médecine, pour formuler des produits dédiés au traitement de l'incontinence). D'autre part, d'un point de vue sécurité, il est important de préciser que cette moins forte diminution du module élastique G' est synonyme d'une dégradation/dénaturation moins importante de l'acide hyaluronique, et donc d'une apparition plus faible des petites masses moléculaires inférieures à 50 000 Da; masses moléculaires étant décrites comme potentiellement pro-inflammatoires et donc susceptibles de générer des effets indésirables.

### Exemple 5 : Préparation d'un gel G6 selon l'invention

On pèse 1 g d'une poudre de hyaluronate de sodium (NaHA), de masse moléculaire environ égale à 1.8 MDa, avec un taux d'humidité de 5.2%, auquel on ajoute 20 g d'une solution de tampon phosphate qui contient également 0.2 mg/g de tréhalose. On mélange manuellement le gel à la spatule pendant 10 minutes puis on mélange à nouveau pendant 10 minutes supplémentaires après avoir laissé le gel s'hydrater pendant 24 heures à la température de 5°C.

Soit G6A le gel ainsi obtenu. La concentration en acide hyaluronique de G6A est de 50 mg/g et la concentration en tréhalose est de 0.2 mg/g (ratio massique [HA]/[TH] égal à 250).

Le gel G6A est conditionné en seringues de 1 ml puis stérilisé à l'autoclave à 121°C pendant 15 minutes. Soit G6 (= gel selon l'invention), le gel stérilisé issus du gel G6A.

## Revendications

1. Composition aqueuse stérile injectable, stérilisée à la chaleur humide, sous forme de gel, comprenant au moins de l'acide hyaluronique réticulé, ou l'un de ses sels, et du tréhalose, **caractérisée en ce que** :
∘ la proportion massique en eau est supérieure à 51% de la masse totale
∘ la proportion massique en tréhalose est supérieure à 0.001% de la masse totale.

2. Composition selon la revendication 1, **caractérisée en ce que** la concentration totale en acide hyaluronique, ou l'un de ses sels, est comprise entre 9 et 30 mg/ml.

3. Composition selon l'une des revendications 1 à 2, **caractérisée en ce que** la proportion massique en eau est supérieure à 80% de la masse totale.

4. Composition selon l'une des revendications 1 à 3, **caractérisée en ce que** la proportion massique en tréhalose est inférieure à 10% de la masse totale.

5. Composition selon l'une des revendications 1 à 4, **caractérisée en ce que** l'acide hyaluronique est réticulé avec le 1,4-butanedioldiglycidyléther (BDDE).

6. Composition selon l'une des revendications 1 à 5, **caractérisée en ce que** la composition contient un agent anesthésique dispersé au sein de l'hydrogel, par exemple de la lidocaïne.

7. Composition selon l'une des revendications 1 à 6, **caractérisée en ce que** le ratio massique entre la concentration massique en acide hyaluronique [HA] et la concentration massique en tréhalose, c'est-à-dire le ratio [HA]/[tréhalose], est compris entre 0.01 et 250.

8. Composition aqueuse stérile injectable, stérilisée à la chaleur humide, sous forme de gel, comprenant au moins de l'acide hyaluronique réticulé, ou l'un de ses sels, **caractérisée en ce que** le réticulant utilisé pour réticuler l'acide hyaluronique, ou l'un de ses sels, est le tréhalose diglycidyl éther (TDGE).

9. Composition selon la revendication 8, **caractérisée en ce que** la composition contient du tréhalose dispersé dans le gel.

10. Composition selon l'une des revendications 8 à 9, **caractérisée en ce que** la proportion massique en tréhalose dispersé dans le gel est inférieure à 10% de la masse totale.

11. Procédé de préparation d'une composition aqueuse stérile injectable comprenant au moins de l'acide hyaluronique réticulé, ou l'un de ses sels, et du tréhalose, **caractérisé en ce qu'**il comprend au moins les étapes successives suivantes :
- préparation d'un gel à base d'acide hyaluronique réticulé, ou l'un de ses sels
- ajout du tréhalose dans le gel avec opération de mélange
- stérilisation par autoclavage.

12. Procédé de préparation d'une composition aqueuse stérile injectable comprenant au moins de l'acide hyaluronique réticulé, ou l'un de ses sels, et du tréhalose, **caractérisé en ce qu'**il comprend au moins les étapes successives suivantes :
- préparation d'un gel à base d'acide hyaluronique réticulé, ou l'un de ses sels, en utilisant un dérivé d'un ose non réducteur transformé au préalable en composé bi- ou poly-fonctionnel par voie chimique ou par toute autre voie, en tant qu'agent de réticulation
- ajout optionnel de tréhalose dans le gel avec opération de mélange
- stérilisation par autoclavage.

13. Utilisation de la composition selon l'une des revendications 1 à 10 pour des applications esthétiques.

14. Composition selon l'une des revendications 1 à 10, pour utilisation dans le cadre d'un traitement thérapeutique.

## Patentansprüche

1. Injizierbare sterile wässrige Zusammensetzung, die mit feuchter Hitze sterilisiert wurde, in der Form von Gel, die mindestens vernetzte Hyaluronsäure oder eines ihrer Salze und Trehalose umfasst, **dadurch gekennzeichnet, dass**:
∘ der Massenanteil an Wasser höher als 51% der Gesamtmasse ist
∘ der Massenanteil an Trehalose höher als 0,001% der Gesamtmasse ist.

2. Zusammensetzung nach Anspruch 1, **dadurch gekennzeichnet, dass** die Gesamtkonzentration an Hyaluronsäure oder einem ihrer Salze zwischen 9 und 30 mg/ml beträgt.

3. Zusammensetzung nach einem der Ansprüche 1 bis 2, **dadurch gekennzeichnet, dass** der Massenanteil an Wasser höher als 80% der Gesamtmasse ist.

4. Zusammensetzung nach einem der Ansprüche 1 bis 3, **dadurch gekennzeichnet, dass** der Massenanteil an Trehalose niedriger als 10% der Gesamtmasse ist.

5. Zusammensetzung nach einem der Ansprüche 1 bis 4, **dadurch gekennzeichnet, dass** die Hyaluronsäure mit 1,4-Butandioldiglycidylether (BDDE) vernetzt ist.

6. Zusammensetzung nach einem der Ansprüche 1 bis 5, **dadurch gekennzeichnet, dass** die Zusammensetzung ein in dem Hydrogel dispergiertes Anästhetikum, zum Beispiel Lidocain, enthält.

7. Zusammensetzung nach einem der Ansprüche 1 bis 6, **dadurch gekennzeichnet, dass** das Massenverhältnis zwischen der Massenkonzentration an Hyaluronsäure [HA] und der Massenkonzentration an Trehalose, das heißt das Verhältnis [HA]/[Trehalose], zwischen 0,01 und 250 beträgt.

8. Injizierbare sterile wässrige Zusammensetzung, die mit feuchter Hitze sterilisiert wurde, in der Form von Gel, die mindestens vernetzte Hyaluronsäure oder eines ihrer Salze umfasst, **dadurch gekennzeichnet, dass** das Vernetzungsmittel, das zum Vernetzen der Hyaluronsäure oder eines ihrer Salze verwendet wird, Trehalose-Diglycidylether (TDGE) ist.

9. Zusammensetzung nach Anspruch 8, **dadurch gekennzeichnet, dass** die Zusammensetzung in dem Gel dispergierte Trehalose enthält.

10. Zusammensetzung nach einem der Ansprüche 8 bis 9, **dadurch gekennzeichnet, dass** der Massenanteil an in dem Gel dispergierter Trehalose niedriger als 10% der Gesamtmasse ist.

11. Verfahren zur Herstellung einer injizierbaren sterilen wässrigen Zusammensetzung, die mindestens vernetzte Hyaluronsäure oder eines ihrer Salze und Trehalose umfasst, **dadurch gekennzeichnet, dass** es mindestens die folgenden aufeinanderfolgenden Schritte umfasst:
- Herstellung eines Gels auf Basis von vernetzter Hyaluronsäure oder eines ihrer Salze
- Zusatz von Trehalose zu dem Gel mit Mischbetrieb
- Sterilisierung durch Behandlung im Autoklav.

12. Verfahren zur Herstellung einer injizierbaren sterilen wässrigen Zusammensetzung, die mindestens vernetzte Hyaluronsäure oder eines ihrer Salze und Trehalose umfasst, **dadurch gekennzeichnet, dass** es mindestens die folgenden aufeinanderfolgenden Schritte umfasst:
- Herstellung eines Gels auf Basis von vernetzter Hyaluronsäure oder eines ihrer Salze unter Verwendung eines Derivats einer nicht reduzierenden Ose, die im Voraus auf chemischem Weg oder auf jedem anderen Weg in eine bi- oder polyfunktionelle Verbindung umgewandelt wurde, als Vernetzungsmittel,
- wahlweise Zusatz von Trehalose zu dem Gel mit Mischbetrieb
- Sterilisierung durch Behandlung im Autoklav.

13. Verwendung der Zusammensetzung nach einem der Ansprüche 1 bis 10 für ästhetische Anwendungen.

14. Zusammensetzung nach einem der Ansprüche 1 bis 10 zur Verwendung im Rahmen einer therapeutischen Behandlung.

## Claims

1. A sterile injectable aqueous composition, sterilized with moist heat, in gel form, comprising at least cross-linked hyaluronic acid, or a salt thereof, and trehalose, **characterized in that**:
∘ the mass proportion of water is greater than 51% of the total mass
∘ the mass proportion in trehalose is greater than 0.001% of the total mass.

2. The composition as claimed in claim 1, **characterized in that** the total concentration of hyaluronic acid, or a salt thereof, is between 9 and 30 mg/ml.

3. The composition as claimed in one of claims 1 to 2, **characterized in that** the mass proportion of water is greater than 80% of the total mass.

4. The composition as claimed in one of claims 1 to 3, **characterized in that** the mass proportion of trehalose is less than 10% of the total mass.

5. The composition as claimed in one of claims 1 to 4, **characterized in that** the hyaluronic acid is crosslinked with 1,4-butanediol diglycidyl ether (BDDE).

6. The composition as claimed in one of claims 1 to 5, **characterized in that** the composition contains an anesthetic agent dispersed within the hydrogel, for example lidocaine.

7. The composition as claimed in one of claims 1 to 6, **characterized in that** the mass ratio between the mass concentration of hyaluronic acid [HA] and the mass concentration of trehalose, i.e. the ratio [HA]/[trehalose], is between 0.01 and 250.

8. A sterile injectable aqueous composition, sterilized with moist heat, in gel form, comprising at least cross-linked hyaluronic acid, or a salt thereof, **characterized in that** the cross-linking agent used to cross-link the hyaluronic acid, or a salt thereof, is trehalose diglycidyl ether (TDGE).

9. The composition as claimed in claim 8, **characterized in that** the composition contains trehalose dispersed in the gel.

10. The composition as claimed in one of claims 8 to 9, **characterized in that** the mass proportion of trehalose dispersed in the gel is less than 10% of the total mass.

11. A process for the preparation of a sterile injectable aqueous composition comprising at least cross-linked hyaluronic acid, or a salt thereof, and trehalose, **characterized in that** it comprises at least the following successive steps:
- preparation of a gel based on cross-linked hyaluronic acid or a salt thereof
- addition of the Trehalose in the gel with mixing operation
- autoclave sterilization.

12. A process for the preparation of a sterile injectable aqueous composition comprising at least cross-linked hyaluronic acid, or a salt thereof, and trehalose, **characterized in that** it comprises at least the following successive steps:
- preparation of a gel based on cross-linked hyaluronic acid, or a salt thereof, using a derivative of a non-reducing monosaccharide first converted into a bi- or poly-functional compound by chemical or other means, as cross-linking agent
- optional addition of trehalose in the gel with mixing operation
- autoclave sterilization.

13. Use of the composition as claimed in one of claims 1 to 10 for aesthetic applications.

14. The composition as claimed in one of claims 1 to 10, for use in therapeutic treatment.
